# EUROPEAN PATENT APPLICATION

(11) **EP 4 799 609 A1**
(43) Date of publication of application: **02.09.2026**
(21) Application number: 23956903.1
(22) Date of filing: 27.10.2023
(51) Int. Cl.: A61K 9/08, A61K 47/02, A61K 47/12, A61K 47/26, A61K 47/18, A61K 38/08, A61P 19/02

(54) **PEPTIDE-CONTAINING LIQUID PREPARATION, AND PREPARATION METHOD THEREFOR**

(71) Applicant: Ensol Biosciences Inc., Daejeon 34036 (KR)
(72) Inventor: KIM, Hae Jin, Daejeon 34023 (KR); LEE, Tai Au, Seoul 02448 (KR); LEE, Cheol Min, Daejeon 35355 (KR); HWANG, Sung Min, Daejeon 34127 (KR); JEON, Mi Ni, Daejeon 34192 (KR); GUK, Seon, Daejeon 34046 (KR); JO, Kang Ick, Daejeon 34048 (KR)
(74) Representative: dompatent
(86) International application number: PCT/KR2023/016934
(87) International publication number: WO 2025/089468

(57) **Abstract**

The present disclosure provides a peptide-containing liquid formulation containing an active ingredient and a buffer solution, wherein the active ingredient is a peptide having an amino acid sequence of SEQ ID NO: 1 or a pharmaceutically acceptable salt thereof, the buffer solution is a phosphoric acid buffer solution, a succinic acid buffer solution, or an acetic acid buffer solution, and the liquid formulation has a pH in the range of 4 to 6, and a method of preparing the same. There is an advantage that the present disclosure can stably formulate the peptide having the amino acid sequence of SEQ ID NO: 1 or the pharmaceutically acceptable salt thereof, even in a liquid phase. In addition, there is an advantage in that the formulation of the present disclosure can be stable at room temperature, even in a liquid phase, thus making formulation into a freeze-dried form unnecessary, can be convenient to store and distribute, and can be easy to prepare and use.

## Description

### Technical Field

The present disclosure relates to a peptide-containing liquid formulation containing a peptide having an amino acid sequence of SEQ ID NO: 1 or a pharmaceutically acceptable salt thereof as an active ingredient and to a method of preparing the same. More particularly, the present disclosure relates to a stable peptide-containing liquid formulation containing a peptide having an amino acid sequence of SEQ ID NO: 1 or a pharmaceutically acceptable salt thereof as an active ingredient and to a method of preparing the same.

### Background Art

The development of pharmaceuticals based on specific peptides or pharmaceutically acceptable salts thereof as active ingredients, in addition to traditional compounds, is in progress (see International Patent Application Publication No. WO 2012/002668 A2). Such peptides are also chemically synthesizable and thus may be viewed as one type of compound. However, peptides have electrochemical properties or the like that differ from the properties of compounds that have been developed as existing pharmaceuticals, so it is difficult to predict the properties of peptides. In particular, unexpected challenges may arise in peptide formulation processes.

The inventors of the present disclosure encountered unexpected challenges in such peptide formulation processes and, while in the process of overcoming such challenges, completed the preset disclosure.

### [Document of related art]

### [Patent Document]

(Patent Document 1) International Patent Application Publication No. WO 2012/002668 A2, 2012.1.5, Specification

### Disclosure

### Technical Problem

One objective of the present disclosure is to provide a stable peptide-containing liquid formulation containing a peptide having an amino acid sequence of SEQ ID NO: 1 or a pharmaceutically acceptable salt thereof as an active ingredient.

In addition, another objective of the present disclosure is to provide a method of preparing a stable peptide-containing liquid formulation containing a peptide having an amino acid sequence of SEQ ID NO: 1 or a pharmaceutically acceptable salt thereof as an active ingredient.

Objectives of the present disclosure are not limited to the above description, and other objectives can be clearly understood by those skilled in the art from the following description.

### Technical Solution

The present disclosure provides a peptide-containing liquid formulation containing an active ingredient and a buffer solution, wherein the active ingredient is a peptide having an amino acid sequence of SEQ ID NO: 1 or a pharmaceutically acceptable salt thereof, the buffer solution is a phosphoric acid buffer solution, a succinic acid buffer solution, or an acetic acid buffer solution, and the liquid formulation has a pH in the range of 4 to 6.

In addition, the present disclosure provides a method of preparing a peptide-containing liquid formulation, the method including: Step (A) of preparing an active ingredient-containing buffer solution; and Step (B) of adjusting the pH of the active ingredient-containing buffer solution to a range of 4 to 6, wherein the active ingredient is a peptide having an amino acid sequence of SEQ ID NO: 1 or a pharmaceutically acceptable salt thereof, and the buffer solution is a phosphoric acid buffer solution, a succinic acid buffer solution, or an acetic acid buffer solution.

### Advantageous Effects

The present disclosure can stably formulate a peptide having an amino acid sequence of SEQ ID NO: 1 or a pharmaceutically acceptable salt thereof, even in a liquid phase. In addition, a formulation of the present disclosure can be stable at room temperature, even in a liquid phase, thus making formulation into a freeze-dried form unnecessary, can be convenient to store and distribute, and can be easy to prepare and use.

### Description of Drawings

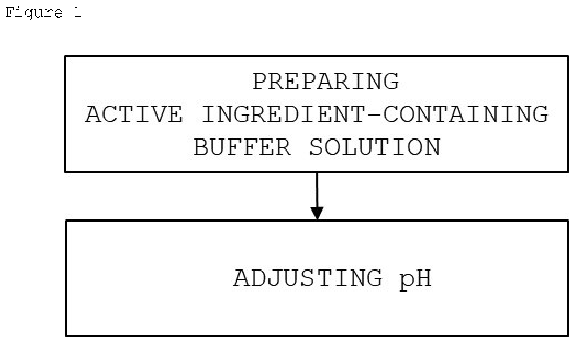

FIG. 1 is a flowchart for describing one embodiment of the present disclosure.

### Mode for Invention

Although the present disclosure will be described in more detail below through embodiments and preparation examples, the following embodiments and preparation examples are only for illustrative purposes of the present disclosure, and the description of the present disclosure is not limited by these embodiments or preparation examples.

A peptide-containing liquid formulation, which is one embodiment of the present disclosure, contains an active ingredient and a buffer solution and has a pH in the range of 4 to 6.

The active ingredient is a peptide having an amino acid sequence (ELHLD) of SEQ ID NO: 1 or a pharmaceutically acceptable salt thereof. In the amino acid sequence, E stands for glutamic acid (Glu), L stands for leucine (Leu), H stands for histidine (His), and D stands for aspartic acid (aspartate, Asp). Peptide-constituting amino acids are present in L-, D-, and DL-forms, and the amino acids constituting the peptide of the present disclosure include all of such forms. In addition, the salt may, for example, be hydrochloride, sulfate, phosphate, acetate, trifluoroacetate, citrate, tartrate, succinate, lactate, maleate, fumarate, oxalate, methanesulfonate, para-toluenesulfonate, a sodium salt, a potassium salt, a magnesium salt, a calcium salt, or the like. The trifluoroacetate may be ditrifluoroacetate (2TFA). Specifically, the active ingredient is preferably trifluoroacetate of the peptide consisting of the amino acid sequence of SEQ ID NO: 1 and is more preferably ditrifluoroacetate of the peptide consisting of the amino acid sequence of SEQ ID NO: 1.

In this case, reference may be made to those described in International Patent Application Publication No. WO 2012/002668 A2 (2012.1.5) for the description of the active ingredients. For example, the active ingredient may show therapeutic and/or preventive effects on cartilage damage and/or arthritis (including osteoarthritis). In addition, the active ingredient or the formulation containing the active ingredient may be for therapeutic and/or preventive use in cartilage damage and/or arthritis (including osteoarthritis). The treatment and/or prevention may be achieved by one or more selected from cartilage tissue regeneration, inhibition of cartilage tissue matrix-degrading enzyme expression, and inhibition of cartilage tissue ossification. Arthritis includes joint diseases involving degeneration of cartilage and subchondral bone.

For animals (including mammals), including humans, the parenteral dose based on the active ingredient when being administered is in the range of 150 µg/day to 1 mg/day and preferably in the range of 0.5 mg/day to 1 mg/day. In the case of oral administration, the dose is 1.2 to 1.5 times as much as the parenteral dose. The active ingredient or the formulation containing the active ingredient is administered in a parenteral manner, for example, by local injection (intra-articular injection), intravenous or subcutaneous injection, or nasal administration. In addition, oral administration may be carried out in some cases.

The active ingredient may be contained in an amount in the range of 0.05% to 1% by weight of the total weight of the formulation.

The buffer solution refers to a solution containing a bufferable component. In this case, the buffer solution is a phosphoric acid buffer solution, a succinic acid buffer solution, or an acetic acid buffer solution.

The phosphoric acid buffer solution may be formed from phosphoric acid and may be formed by applying a phosphoric acid-based buffer (for example, monosodium phosphate, disodium phosphate, and the like).

In addition, the acetic acid buffer solution may be formed from acetic acid and may be formed by applying an acetic acid-based buffer (for example, sodium acetate, acetic acid, and the like).

Furthermore, the succinic acid buffer solution may be formed from succinic acid and may be formed by applying a succinic acid-based buffer (for example, sodium succinate, succinic acid, and the like).

The amount based on phosphate, succinate, or acetate contained respectively in the phosphoric acid buffer solution, the succinic acid buffer solution, or the acetic acid buffer solution is preferably in the range of 1 to 1000 parts by weight, more preferably in the range of 5 to 700 parts by weight, and even more preferably in the range of 10 to 500 parts by weight, with respect to 100 parts by weight of the active ingredient.

When the formulation, which is one embodiment of the present disclosure, contains such a buffer solution and has a pH in the range of 4 to 6, the active ingredient can be stable, especially in the liquid phase.

In this case, the term "stable" means that the pyro-form impurity (related substances) content in the active ingredient is 3% or less. In addition, in the formulation, which is one embodiment of the present disclosure, the active ingredient preferably has a purity of 95% or more. In the process of completing the present disclosure, the pyro-form impurities (related substances) generated in the active ingredient were recognized, and such generation was inhibited by the present disclosure. The amount of present impurities (related substances) can be measured by high-performance liquid chromatography (HPLC)-analysis. Changes in the amount of related substances can be calculated as the sum of the area ratios of the total amount of related substances in the formulation to be analyzed. According to the present disclosure, the generation of impurities (related substances) can be inhibited, and the liquid phase can be maintained in a stable state. In particular, the stability can be maintained in the liquid phase, even at room temperature. Thus, according to the present disclosure, formulation into a freeze-dried form is unnecessary. In other words, storage and distribution do not need to be carried out in a refrigerated state, making the use of the formulation easier in areas where refrigerated storage is not easily available. In terms of field application, the liquid phase is available as it is without involving a process of turning a freeze-dried product into a liquid as an injectable, which is convenient. In addition, a freeze-drying process is unnecessary during the preparation process, so the preparation is simplified. The room temperature, in this case, refers to a range that is preferably in the range of 5°C to 40°C, more preferably in the range of 10°C to 30°C, and even more preferably in the range of 23°C to 27°C.

In the meantime, in one embodiment, the pH may be adjusted using a pH adjuster. The pH adjuster is not limited to as long as it is capable of adjusting pH but may, for example, be one or more selected from sodium hydroxide, potassium hydroxide, sodium hydrogen carbonate, sodium carbonate, potassium hydrogen carbonate, potassium carbonate, acetic acid, or hydrochloric acid. The pH adjuster may be used in an amount that is preferably in the range of 1 to 1000 parts by weight, more preferably in the range of 5 to 700 parts by weight, and even more preferably in the range of 10 to 500 parts by weight, with respect to 100 parts by weight of the active ingredient. Below or above such a range, there is a concern that the formulation is unstable.

In addition, one embodiment may further include a pharmaceutically acceptable additive. The additive may, for example, be one or more selected from sodium chloride, sorbitol, or glycine. The additive may be used in a pharmaceutically acceptable amount, which is preferably in the range of 1 to 10000 parts by weight, more preferably in the range of 100 to 8000 parts by weight, and even more preferably in the range of 500 to 6000 parts by weight, with respect to 100 parts by weight of the active ingredient.

With the addition of such an additive, the formulation may become further stable.

The additive is more preferably combined depending on the buffer solution. Specifically, in one embodiment of the present disclosure, the buffer solution is the acetic acid buffer solution, the additive is one or more selected from sodium chloride or sorbitol, and the pH is in the range of 4 to 5.5; the buffer solution is the succinic acid buffer solution, the additive is glycine, and the pH is in the range of 5 to 6; or the buffer solution is the phosphoric acid buffer solution, the additive is one or more selected from sodium chloride or sorbitol, and the pH is in the range of 5.5 to 6.

The liquid formulation, which is one embodiment of the present disclosure, may be prepared by a preparation method, which is one embodiment of the present disclosure. Hereinafter, a method of preparing a peptide-containing liquid formulation, which is one embodiment of the present disclosure, will be described in more detail with reference to FIG. 1.

FIG. 1 is a flowchart for describing one embodiment of the present disclosure. As shown in FIG. 1, the method of preparing the peptide-containing liquid formulation, which is one embodiment of the present disclosure, includes: Step (A) of preparing an active ingredient-containing buffer solution; and Step (B) of adjusting the pH.

Step (A) of preparing the active ingredient-containing buffer solution is to prepare an active ingredient-containing buffer solution. The active ingredient and the buffer solution are practically in the same range as those of the formulation, which is one embodiment of the present disclosure. However, the buffer solution in Step (A) refers to a solution containing a component (for example, buffers) capable of forming the buffer solution regardless of its actual buffering capacity. In other words, the buffer solution in Step (A) is not limited to those exhibiting buffering capacity all the time in all steps as long as it serves as at least one component that imparts buffering capacity to the final liquid formulation.

The buffer solution may further include an additive, and this additive is also practically in the same range as that of the formulation, which is one embodiment. Specifically, in Step (A), the buffer solution may be formed by mixing an additive-containing aqueous solution with the component capable of forming the buffer solution. The additive and the component (for example, buffers) capable of forming the buffer solution can form a mixture, regardless of order. For example, an aqueous solution containing a pharmaceutically effective amount of the component (for example, buffers) capable of forming the buffer solution may first be prepared, followed by adding the additive. Alternatively, the additive may be dissolved in water, followed by adding a pharmaceutically effective amount of the component (for example, buffers) capable of forming the buffer solution. The buffer may, for example, be acetic acid, phosphoric acid, succinic acid, monopotassium phosphate, dipotassium phosphate, sodium acetate, disodium phosphate, monosodium phosphate, and/or sodium succinate. In this case, the active ingredient may also be added to water in conjunction with the additive and/or the component capable of forming the buffer solution, regardless of order, and mixed. In this case, a basifying agent, which is a strong base (for example, sodium hydroxide), can be added to the buffer, which is a weak acid, to form the buffer solution. In addition, the pH may be adjusted before adding the active ingredient. The pH adjustment may be carried out through a process of adding the basifying agent (for example, sodium hydroxide) to water. Such a basifying agent can serve a buffer solution-forming role in conjunction with the weak acid.

Step (B) of adjusting the pH is to adjust the pH of the active ingredient-containing buffer solution to a range of 4 to 6. The adjustment in Step (B) may be carried out using a pH adjuster, and the pH adjuster is practically in the same range as that of the formulation, which is one embodiment of the present disclosure.

Through these steps, a stable peptide-containing liquid formulation, which is one embodiment of the present disclosure, can be prepared.

Unless otherwise mentioned, those mentioned in the formulation and the preparation method of the present disclosure are applied to each other to the extent of identity if not in contradiction with each other.

Through the following preparation example and reference example, the preparation method and the peptide, each of which is an embodiment of the present disclosure, will be explained in more detail.

Reagents and materials used in the following preparation example and reference example, being currently available in the market and of the best quality, were purchased from Sigma-Aldrich unless otherwise mentioned.

### <Example> Preparation of peptide-containing liquid formulation

### 1.1. Preparation of peptide prepared by solid-phase peptide preparation method

The trifluoroacetate (Glu-Leu-His-Leu-Asp·2TFA) of a peptide having an amino acid sequence listed in Table 1 was prepared by a common solid-phase peptide preparation method. Specifically, the preparation was carried out by a standard fluorenylmethyloxycarbonyl solid-phase peptide synthesis (Fmoc-SPPS) method using a starting material, an H-Asp(OtBu)-2-ClTrt solid resin (substitution rate: 0.63 to 0.67 mmol/g). First, 125.0 g of the H-Asp(OtBu)-2-ClTrt peptide resin, serving as the starting material, was introduced into a welldried reactor, followed by introducing N,N-dimethylformamide (DMF, 1250 ml, 10 mL/g resin). The resulting resin was swollen while being stirred for 20 minutes, followed by filtration to discard the resulting filtrate. Then, the remaining resin was washed twice with N,N-dimethylformamide (DMF, 750 ml, 6 mL/g resin). Into another reactor, Fmoc-Leu-OH (1.5 equivalents with respect to that of the starting material) and hydroxybenzotriazole (HOBt, 1.5 equivalents with respect to that of the starting material) were introduced, followed by introducing N,N-dimethylformamide (DMF, 4.5 mL per gram of the starting material) for dissolution. Then, diisopropylethylamine (DIPEA, 1.5 equivalents with respect to that of the starting material) was added. The reaction solution was cooled to a temperature in the range of 0°C to 5°C, and 3-[bis(dimethylamino)methyliumyl]-3H-benzotriazole-1-oxide hexafluorophosphate (HBTU, 1.5 equivalents with respect to that of the starting material) was then added, thereby activating the carboxyl group of the amino acid. Dichloromethane (1.5 mL per 1 g of the starting material) was added to the reactor containing the peptide resin, serving as the starting material, and then the resulting product was stirred while slowly introducing the solution of the activated amino acid (Fmoc-Leu-OH) derivative prepared in another reactor. Then, the reaction solution was stirred for 2 hours at a temperature in the range of 10°C to 30°C. The reaction was confirmed using the ninhydrin (Kaiser) test. After completion of the reaction, the peptide-resin was filtered, followed by washing with respective solvents, N,N-dimethylformamide (DMF) and methyl t-butyl ether (MTBE). Then, the resulting peptide-resin solid was washed again twice with N,N-dimethylformamide (DMF) (750 ml, 6 mL/g resin). For the following fluorenylmethyloxycarbonyl (Fmoc) deprotection reaction, the peptide-resin was treated with a 5% piperidine mixed solution (v/v/w/v: 5% piperidine/1.25% DBU/1% HOBt/DMF) twice (750 ml, 6 mL/g resin, stirred for 10 minutes in each time and then filtered). After conducting the deprotection reaction, the resulting product was filtered. Then, the peptide-resin obtained through the filtration was washed with respective solvents, N,N-dimethylformamide (DMF), methyl t-butyl ether (MTBE), and N,N-dimethylformamide (DMF) (60 mL/g resin for each solvent), twice. The fluorenylmethyloxycarbonyl (Fmoc) deprotection reaction was confirmed using the ninhydrin (Kaiser) test. Coupling and fluorenylmethyloxycarbonyl (Fmoc) deprotection reactions for #3, #4, and #5 amino acid derivatives were carried out in the same manner as described above. In other words, amino acids were sequentially reacted with the peptide-resin using #3 reaction Fmoc-His(Trt)-OH, #4 reaction Fmoc-Leu-OH, and #5 reaction Fmoc-Glu(OtBu)-OH, thereby synthesizing an Fmoc-Glu(OtBu)1-Leu2-His(Trt)3-Leu4-Asp(OtBu)5-2-ClTrt peptide-resin. However, in the case where the #3 amino acid introduces Fmoc-His(Trt)-OH, the coupling reaction was carried out using mixed solvents, N,N-dimethylformamide/dichloromethane (DCM/DMF) and N-hydroxybenzotriazole/N,N,N-diisopropylcarbodiimide for carboxyl group activation. A 5% piperidine mixed solvent (v/v/w/v: 5% piperidine/1.25% DBU/1% HOBt/DMF) was added to the N-terminal fluorenylmethyloxycarbonyl (Fmoc) group of the Fmoc-Glu(OtBu)1-Leu2-His(Trt)3-Leu4-Asp(OtBu)5-2-ClTrt peptide-resin, followed by stirring, filtration, and washing, thereby synthesizing an H-Glu(OtBu)1-Leu2-His(Trt)3-Leu4-Asp(OtBu)5-2-ClTrt peptide-resin.

Into another reactor, a mixed solution of trifluoroacetic acid/triisopropylsilane/dichloromethane (TFA/TIS/DCM: 55/5/40, v/v/v, 450 ml, 10 mL per 1 g of the peptide resin), serving as a deprotection mixed solution, was introduced, and then the resulting product was cooled to a temperature in the range of 0°C to 5°C. The obtained peptide-resin was slowly added to the cooled solution while maintaining the temperature of the reaction solution to lower than 17°C (titration temperature, 13.8°C to 14.6°C). Subsequently, the reaction solution was heated to a temperature in the range of 23°C to 27°C and stirred at the same temperature for 90 minutes. The resulting reaction solution was filtered using a polytetrafluoroethylene (PTFE) filter to obtain a solid resin, followed by washing twice with trifluoroacetic acid (TFA) (1.0 mL per gram of the peptide resin twice) to collect all the filtrate. Next, the collected filtrate was concentrated under reduced pressure at a temperature of 30°C or lower until the amount thereof became 20% to 30% by volume. A pre-cooled methyl t-butyl ether (MTBE) (about 10 times as much as the concentration volume) serving as a solvent was added to the concentrated residues to produce a crude peptide as a solid precipitate. The solid precipitate produced was stirred for 1 hour at room temperature, followed by filtration and washing five times with cooled methyl t-butyl ether (MTBE) (50% of the amount of MTBE used for precipitation) serving as a solvent. The crude peptide obtained through the filtration was vacuum-dried at a temperature of 24°C for about 30 hours, thereby synthesizing a ditrifluoroacetate (Glu-Leu-His-Leu-Asp·2TFA, 57.4 g) of a crude peptide having an amino acid sequence of SEQ ID NO: 1, the crude peptide having a deprotection peptide yield of 98.4% and an HPLC purity of 86%. Then, the ditrifluoroacetate of the crude peptide was separated by a reversed-phase high-performance liquid chromatography method, using a purified water/acetonitrile development solvent system containing 0.1% (v/v) trifluoroacetic acid. The purity (listed in Table 1, 97% or more) and the molecular weight of the purified peptide (Glu-Leu-His-Leu-Asp·2TFA) were confirmed using liquid chromatography/mass spectrometry (LC/MS). The purity and the TFA equivalent were confirmed using an HPLC method, and the amino acid sequence was confirmed using a liquid chromatography-tandem mass spectrometry (LC/MS/MS) method. In addition, the molecular weight was confirmed using an LC/MS method. The results thereof are shown in Table 1 below.

**[Table 1]**

| | Amino acid sequence of peptide | Purity (%) | TFA equivalent | Mass analyzer | |
|---|---|---|---|---|---|
| | | | | Theoretical value | Analysis value |
| 1-1 | Glu-Leu-His-Leu-Asp (SEQ ID NO: 1) | 97% or more | 2 | 625.7 | 625.4 |

### 1.2. Preparation of active ingredient-containing buffer solution and pH adjustment

Buffers and additives listed in Table 2 and 3600 ml of water for injection were introduced into a well-washed reactor. The resulting product was stirred for 10 minutes at room temperature (25°C), and then a 1 M sodium hydroxide solution was slowly added dropwise to adjust the pH of the reaction solution to 5.5, followed by stirring for 30 minutes. At the same temperature, 3.6 g of the peptide salt (1-1) prepared in 1.1. was introduced, and then pH adjusters were slowly added dropwise to adjust the pH of the solution to values listed in Table 2. The dissolved solution was filtered through a 0.2 microfilter, and a vial or syringe was filled with the resulting product.

**[Table 2]**

| | Buffer | Additive | pH adjuster | pH |
|---|---|---|---|---|
| 1-2-1 | Sodium acetate (2.07 g) | Sodium chloride (32.4 g) | Hydrochloric acid | 4.0 |
| | Acetic acid (0.64 g) | | | |
| 1-2-2 | Sodium acetate (2.07 g) | Sorbitol (180 g) | Hydrochloric acid | 4.0 |
| | Acetic acid (0.64 g) | | | |
| 1-2-3 | Sodium acetate (2.07 g) | Sodium chloride (32.4 g) | Hydrochloric acid | 5.0 |
| | Acetic acid (0.64 g) | | | |
| 1-2-4 | Sodium acetate (2.07 g) | Sorbitol (180 g) | Hydrochloric acid | 5.0 |
| | Acetic acid (0.64 g) | | | |
| 1-2-5 | Succinic acid (4.25 g) | Glycine (72 g) | Sodium hydroxide | 5.5 |
| 1-2-6 | Disodium phosphate (1.32 g) | Sodium chloride (32.4 g) | Sodium hydroxide | 6.0 |
| | Monosodium phosphate (4.29 g) | | | |
| 1-2-7 | Disodium phosphate (1.32 g) | Sorbitol (180 g) | Sodium hydroxide | 6.0 |
| | Monosodium phosphate (4.29 g) | | | |

### <Reference Example> Preparation of peptide-containing liquid formulation

Peptide-containing liquid formulations were prepared in the same manner as in the example, except that the active ingredient-containing buffer solution preparation and the pH adjustment conditions followed conditions listed in Table 3 instead of Table 2.

**[Table 3]**

| | Buffer | Additive | pH adjuster | pH |
|---|---|---|---|---|
| R1-2-1 | - | Sodium chloride (32.4 g) | - | 3.0 |
| R1-2-2 | Sodium acetate (2.07 g) | Sodium chloride (32.4 g) | Hydrochloric acid | 3.5 |
| | Acetic acid (0.64 g) | | | |
| R1-2-3 | Disodium phosphate (1.32 g) | Sodium chloride (32.4 g) | Sodium hydroxide | 6.5 |
| | Monosodium phosphate (4.29 g) | | | |
| R1-2-4 | Disodium phosphate (1.32 g) | Sorbitol (180 g) | Sodium hydroxide | 7.0 |
| | Monosodium phosphate (4.29 g) | | | |
| R1-2-5 | Disodium phosphate (1.32 g) | Sorbitol (180 g) | Sodium hydroxide | 8.0 |
| | Monosodium phosphate (4.29 g) | | | |

### <Experimental Example> Stability test

Stability tests were carried out on the liquid formulations prepared in the example and the reference example. Specifically, the liquid formulations of the example and reference example were stored in a storage chamber under storage conditions {accelerated (40 ± 2°C, relative humidity (RH) = 75 ± 5%)} to confirm the purity and related substances (pyro-form impurities of the active ingredient) content using an HPLC method. Under accelerated 6-month storage conditions, the test results in summary are as shown in Table 4 below.

**[Table 4]**

| | pH | Purity% (HPLC) | Pyro-form impurity% (HPLC) |
|---|---|---|---|
| R1-2-1 | 3.0 | 90.4 | 7.8 |
| R1-2-2 | 3.5 | 94.1 | 3.5 |
| 1-2-1 | 4.0 | 96.9 | 1.4 |
| 1-2-2 | 4.0 | 96.4 | 1.5 |
| 1-2-3 | 5.0 | 96.8 | 1.3 |
| 1-2-4 | 5.0 | 97.1 | 1.1 |
| 1-2-5 | 5.5 | 96.2 | 1.4 |
| 1-2-6 | 6.0 | 95.6 | 2.7 |
| 1-2-7 | 6.0 | 95.7 | 2.5 |
| R1-2-3 | 6.5 | 93.1 | 4.1 |
| R1-2-4 | 7.0 | 92.0 | 6.3 |
| R1-2-5 | 8.0 | 93.5 | 4.8 |

As shown in Table 4, it is seen that the stability of the liquid formulations of the present disclosure is excellent. In particular, when the buffer and the pH adjuster are not applied, the pyro-form impurity content is high at 7.8%, but according to the present disclosure, the pyro-form impurity content is low, showing that the effect is excellent. In addition, in the case of the reference example in which the pH does not fall within the range of 4 to 6, the pyro-form impurity content exceeds 3%, and the purity is not even close to 95%, which is beyond the range of quality control. However, in the case of the example of the present disclosure, all the quality control standards (a pyro-form impurity content of 3% or less and a purity of 95% or more) are met. Thus, it is seen that the example of the present disclosure is a stable liquid formulation that satisfies all the quality control standards. Such an effect is shown when the buffer solution is a phosphoric acid buffer solution, a succinic acid buffer solution, or an acetic acid buffer solution, which is shown to be even more desirable in the case of the succinic acid buffer solution or the acetic acid buffer solution.

Thus, it is seen that according to the present disclosure, the peptide having an amino acid sequence of SEQ ID NO: 1 or the pharmaceutically acceptable salt thereof can be stably formulated, even in a liquid phase. In addition, it is seen that the formulation of the present disclosure can be stable at room temperature, even in a liquid phase, thus making formulation into a freeze-dried form unnecessary, can be convenient to store and distribute, and can be easy to prepare and use.

### Industrial Applicability

The present disclosure can stably formulate a peptide having an amino acid sequence of SEQ ID NO: 1 or a pharmaceutically acceptable salt thereof, even in a liquid phase. In addition, a formulation of the present disclosure can be stable at room temperature, even in a liquid phase, thus making formulation into a freeze-dried form unnecessary, can be convenient to store and distribute, and can be easy to prepare and use. Thus, the present disclosure is industrially applicable.

## Claims

1. A peptide-containing liquid formulation comprising:
an active ingredient; and
a buffer solution,
wherein the active ingredient is a peptide comprising an amino acid sequence of SEQ ID NO: 1 or a pharmaceutically acceptable salt thereof,
the buffer solution is a phosphoric acid buffer solution, a succinic acid buffer solution, or an acetic acid buffer solution, and
the liquid formulation has a pH in a range of 4 to 6.

2. The liquid formulation of claim 1, further comprising one or more additives selected from sodium chloride, sorbitol, or glycine.

3. The liquid formulation of claim 2, wherein the buffer solution is the acetic acid buffer solution, the additive is one or more selected from sodium chloride or sorbitol, and the pH is in a range of 4 to 5.5.

4. The liquid formulation of claim 2, wherein the buffer solution is the succinic acid buffer solution, the additive is glycine, and the pH is in a range of 5 to 6.

5. The liquid formulation of claim 2, wherein the buffer solution is the phosphoric acid buffer solution, the additive is one or more selected from sodium chloride or sorbitol, and the pH is in a range of 5.5 to 6.

6. A method of preparing a peptide-containing liquid formulation, the method comprising:
(A) preparing an active ingredient-containing buffer solution; and
(B) adjusting a pH of the active ingredient-containing buffer solution to a range of 4 to 6,
wherein the active ingredient is a peptide comprising an amino acid sequence of SEQ ID NO: 1 or a pharmaceutically acceptable salt thereof, and
the buffer solution is a phosphoric acid buffer solution, a succinic acid buffer solution, or an acetic acid buffer solution.
